# EUROPEAN PATENT APPLICATION

(11) **EP 4 516 290 A1**
(43) Date of publication of application: **05.03.2025**
(21) Application number: 23194457.0
(22) Date of filing: 31.08.2023
(51) Int. Cl.: A61K 9/16, A61K 31/137, A61K 31/167, A61K 31/192, A61K 45/06

(54) **PHARMACEUTICAL COMPOSITION OF COMBINATION OF A NON-STEROIDAL ANTI-INFLAMMATORY DRUGS (NSAIDS) AND DECONGESTANT IN THE FORM OF A POWDER OR GRANULES FOR PREPARING A HOT DRINK**

(71) Applicant: Grindeks, a joint stock company, 1057 Riga (LV)
(72) Inventor: The designation of the inventor has not yet been filed

(57) **Abstract**

Pharmaceutical composition containing combination of a non-steroidal antiinflammatory drugs (NSAID_{S}) and of an effective amount decongestant and further comprising suitable pharmaceutically acceptable carriers or excipients in the form of powder or granules for making hot drink solution with water.

## Description

### Technical Field

The present invention relates to pharmaceutical composition containing as pharmaceutically active compounds a combination of a non-steroidal anti-inflammatory drugs (NSAIDs) selected from the salts of ibuprofen include the alkali metal salts such as the sodium and potassium salts; the amino acid salts such as the lysine or arginine salts, or amine salts such as the meglumine salt or a pharmaceutically acceptable salt thereof and of an effective amount decongestant selected from the group pseudoephedrine, phenylpropanolamine and phenylephrine or a pharmaceutically acceptable salt thereof and further comprising suitable pharmaceutically acceptable carriers or excipients, a process for it preparation and a palatable formulation thereof suitable for oral consumption. In particular, the invention relates to pharmaceutical composition containing as pharmaceutically active compounds a combination of a non-steroidal anti-inflammatory drugs (NSAIDs) and of an effective amount decongestant in the form of powder or granules, for administration in soluble form after dissolving in hot water and with an acceptable taste, e.g., formulation in aqueous solution as a hot drink.

### Background Art

The most common and preferred route of drug administration is through the oral route. Tablets and capsules are the most popular solid dosage forms. However, difficulty swallowing tablets and capsules can be a problem for many individuals and can lead to a variety of adverse events and patient noncompliance with treatment regimens. It is estimated that over 16 million people in the United States have some difficulty swallowing, also known as dysphagia. For these individuals, swallowing a tablet or a capsule can be particularly challenging. Additionally, sore throat and difficulty swallowing is a common symptom experienced by patients with cold and flu.

Tablets and Capsules are often too big, more than 10 mm, become stuck in the throat and have an unpleasant taste/odour, causing people to chew or crush and dissolve their medication, some even stop taking their medication altogether. User-friendly dosage forms, such as orally powder or granules overcome these problems, as they are easy to swallow and it can be administered easily to patients having difficulty in swallowing like elderly, stroke victims, and paediatrics and have a pleasant taste. Moreover, this increases the bedridden patient's compliance, people travelling having less access to water.

Granules or powders that are packaged into small sachets called 'stick packs' or pouches. Powders and granules for reconstitution are produced as single-dose sachets. Granules or powders are used to fill the final unit dosage package, which must provide light and moisture protection for the drug granules. One example of suitable packaging is foil (for example aluminium) 'stick packs', pouch or sachet.

Whilst capsules and tablets are more convenient for many customers, hot drink remedies are associated with greater comfort, and they provide active ingredients in solution that may result in them reaching the bloodstream and being bioavailable faster than tablet formulations, because the hot beverage allows rapid gastric emptying, in which the food, especially sugar, moves from your stomach into your small bowel quickly. This reduces the time taken between drug ingestion and the onset of symptom control. Lee Ann Hodges, et al. Does a Hot Drink Provide Faster Absorption of Paracetamol Than a Tablet? A Pharmacoscintigraphic Study in Healthy Male Volunteers Pharm Res. 2014; 31(8); p. 2078-2085.

Additionally hot drinks are a common treatment for common cold and flu but there are no studies reported in the scientific and clinical literature on this mode of treatment. This study investigated the effects of a hot fruit drink on objective and subjective measures of nasal airflow, and on subjective scores for common cold/flu symptoms in 30 subjects suffering from common cold/flu. The results demonstrate that the hot drink had no effect on objective measurement of nasal airflow, but it did cause a significant improvement in subjective measures of nasal airflow. The hot drink provided immediate and sustained relief from symptoms of runny nose, cough, sneezing, sore throat, chilliness, and tiredness, whereas the same drink at room temperature only provided relief from symptoms of runny nose, cough, and sneezing. The effects of the drinks are discussed in terms of a placebo effect and physiological effects on salivation and airway secretions. In conclusion the results support the folklore that a hot tasty drink is a beneficial treatment for relief of most symptoms of common cold and flu. A Sanu, et al, The effects of a hot drink on nasal airflow and symptoms of common cold and flu Rhinology, 2008 Dec; 46(4); p.271-5.

Health professionals have long been co-prescribing a non-steroidal anti-inflammatory drug (NSAIDs), like ibuprofen together and another active ingredient, like decongestant therefore there is precedent for additional formulations of fixed dose combinations products to improve availability of strong pain relief to those where a hot drink formulation may be preferable, and for better pain management of the symptoms of cold and flu.

ibuprofen, namely 2-(4-isobutylphenyl) propionic acid, is a well-known analgesic, anti-inflammatory and antipyretic agent. The acid form of ibuprofen is poorly soluble in water (less than 1 mg/ml) and bitter taste. Ibuprofen per se is poorly water-soluble drug having melting point 75-77°C.

ibuprofen composition adapted to form drink products on the addition of hot water have previously been proposed. For example, WO93/20850A1 (SMITHKLINE BEECHAM PLC), 28.10.1993 discloses compositions to which hot water may be added. However, this disclosure requires significant formulation steps to prepare a soluble ibuprofen-beta-cyclodextrin complex which dissolves on the addition of water.

Benefit of ibuprofen containing in powder or granule form is that patients who have difficulties swallowing medicines for a variety of reasons could benefit from taking the same active ingredients and same equivalent doses in a hot drink formulation.

Unlike conventional tablet formulations where consumers will often use 2 tablets, we expect that consumers are only likely to use a single sachet per cup of hot water.

The medicines containing ibuprofen lysine which is the lysine salt of ibuprofen. Ibuprofen is one of a group of non-steroidal anti-inflammatory drugs (known as NSAIDs) which work to reduce pain, fever and swelling and used to relieve headache, migraine, rheumatic and muscular pain, backache, neuralgia (nerve pain), dental pain, period pain, fever (high temperature), the symptoms of cold and flu.

Decongestant selected from the group pseudoephedrine, phenylpropanolamine and phenylephrine or a pharmaceutically acceptable salt thereof are widely used in rhinitis drugs for internal use because they stimulate α-receptors, contract vascular smooth muscle of the nasal mucosa, and reduce blood flow, thereby reducing congestion and swelling of the nasal mucosa and exhibiting a strong nasal congestion improvement effect in other words is used for nasal mucosa vasoconstrictor like relieving congestion of nasal sinuses and nasal mucosa blood vessels, and relieving nasal obstruction symptoms.

Phenylephrine or a pharmaceutically acceptable salt and pseudoephedrine or a pharmaceutically acceptable salt are used to relieve nasal discomfort caused by colds, allergies, and hay fever. It is also used to relieve sinus congestion and pressure. Phenylephrine will relieve symptoms but will not treat the cause of the symptoms or speed recovery. Pseudoephedrine It works by causing narrowing of the blood vessels in the nasal passages.

The product is to be taken by dissolving the powder in hot water and then consumed immediately. The correct dose is included within each sachet and therefore no additional tools are required to ensure the correct dose is dissolved.

Therefore, this hot drink formulation provides an additional benefit of providing these consumers with a more accessible medication formulation at the equivalent dosage of a full dose of tablets, without exceeding the recommended individual dose, or maximum daily dose.

Additionally compared to a standard form tablet, a hot drink provides faster absorption of active ingredients potentially due to more rapid gastric emptying.

### Summary of invention

The powder of powder or granule of a non-steroidal anti-inflammatory drugs (NSAIDs) selected from the salts of ibuprofen include the alkali metal salts such as the sodium and potassium salts; the amino acid salts such as the lysine or arginine salts, or amine salts such as the meglumine salt or a pharmaceutically acceptable salt thereof and of an effective amount decongestant selected from the group pseudoephedrine, phenylpropanolamine and phenylephrine or a pharmaceutically acceptable salt is a new preparation formulation in the pharmaceutical field, has the characteristics and advantages of solid preparations and liquid preparations, is particularly suitable for children, old people and patients who cannot swallow the solid preparations, and has the characteristics of quick response and high bioavailability because the granule is finally taken in a liquid form.

Disclosed double action combined pharmaceutical composition on the one hand effect of a non-steroidal anti-inflammatory drugs (NSAIDs) selected from the salts of ibuprofen include the alkali metal salts such as the sodium and potassium salts; the amino acid salts such as the lysine or arginine salts, or amine salts such as the meglumine salt or a pharmaceutically acceptable salt and on the other hand an effective amount decongestant selected from the group pseudoephedrine, phenylpropanolamine and phenylephrine or a pharmaceutically acceptable salt thereof is used for nasal mucosa vasoconstrictor like relieving congestion of nasal sinuses and nasal mucosa blood vessels, and relieving nasal obstruction symptoms.

Compared to the state of the art, the cold-like symptoms treating with pharmaceutical composition, as disclosed above in the form powder or granules, for administration in soluble form after dissolving in hot water (in the range of 80-100°C) and with an acceptable taste, e.g. formulation in aqueous solution as a hot drink has the advantages that the cold-like symptoms are simultaneously treated, the treatment course is shorter and more effective.

The term "cold-like" symptoms as used herein refers to a blocked or runny nose, sore or scratchy throat, headaches, muscle aches, coughs, sneezing, a raised temperature, low-grade fever, pressure in your ears and face, runny or stuffy nose and generally feeling unwell.

Thus, this pharmaceutical composition has an advantage by having dual action effect and be provided as powder or granules, for administration in soluble form after dissolving in hot water (in the range of 80-100°C) and with an acceptable taste, e.g., formulation in aqueous solution as a hot drink.

Disclosed pharmaceutical composition containing as pharmaceutically active compounds a combination of a non-steroidal anti-inflammatory drugs (NSAIDs) selected from the salts of ibuprofen include the alkali metal salts such as the sodium and potassium salts; the amino acid salts such as the lysine or arginine salts, or amine salts such as the meglumine salt or a pharmaceutically acceptable salt thereof and of an effective amount decongestant selected from the group pseudoephedrine, phenylpropanolamine and phenylephrine or a pharmaceutically acceptable salt thereof and further comprising suitable pharmaceutically acceptable carriers or excipients, a process for it preparation and a palatable formulation thereof suitable for oral consumption. In particular, the invention relates to pharmaceutical composition containing as pharmaceutically active compounds a combination of a non-steroidal anti-inflammatory drugs (NSAIDs) and of an effective amount decongestant in the form of powder or granules, for administration in soluble form after dissolving in hot water and with an acceptable taste, e.g., formulation in aqueous solution as a hot drink.

The invention also describes a process for the manufacture of the pharmaceutical composition of ibuprofen include the alkali metal salts such as the sodium and potassium salts; the amino acid salts such as the lysine or arginine salts, or amine salts such as the meglumine salt or a pharmaceutically acceptable salt thereof and of an effective amount decongestant selected from the group pseudoephedrine, phenylpropanolamine and phenylephrine or a pharmaceutically acceptable salt in the form of powder or granule for oral solution in sachet.

Additionally hot aqueous solution provides has a soothing effect.

### Detailed description of the invention

It has been a particular problem to formulate product containing non-steroidal anti-inflammatory drugs (NSAIDs), ibuprofen include the alkali metal salts such as the sodium and potassium salts; the amino acid salts such as the lysine or arginine salts, or amine salts such as the meglumine salt or a pharmaceutically acceptable salt thereof and decongestant capable of producing an aqueous drink preparation in combination with hot water - in the range of 80-100°C, at the temperature above the melting point of ibuprofen, e.g. 75-77°C.

To solve above mentioned problems different attempts have been made to hide the taste and to improve its solubility, was overcome use the salts of ibuprofen include the alkali metal salts such as the sodium and potassium salts; the amino acid salts such as the lysine or arginine salts, or amine salts such as the meglumine salt or a pharmaceutically acceptable salt thereof, preferably lysine salt of ibuprofen. Following oral administration, ibuprofen lysine dissociates to ibuprofen acid and lysine by having melting temperature above temperature of hot water - in the range of 80-100°C.

ibuprofen L-lysine has melting point 176-179°C, ibuprofen D, L-lysine has melting point 180-182°C and ibuprofen L-arginine 167-170°C.

Lysine has no recognized pharmacological activity. Ibuprofen is more rapidly adsorbed from the Gl tract though the rate of extent of ibuprofen absorption may reduce by food. Most preparations contain racemic mixture of R- (-)- and S- (+)-ibuprofen, with the S- (+) enantiomer possessing the majority of pharmacological activity. US4279926A (PRODOTTI ANTIBIOTICI SPA), 21.07.1981 describes the use of lysine salt of ibuprofen is relieving pain and inflammatory conditions in warm-blooded animals. In the patent US4279926A (PRODOTTI ANTIBIOTICI SPA), 21.07.1981 disclosed D, L-lysine salt of 2-(4-isobutyl-phelyl)-propionic acid in the form of a colourless, crystalline substance with the melting point 176-177°C.

Thus, non-steroidal anti-inflammatory drugs (NSAIDs), ibuprofen include the alkali metal salts such as the sodium and potassium salts; the amino acid salts such as the lysine or arginine salts, or amine salts such as the meglumine salt or a pharmaceutically acceptable salt thereof and decongestant composition is required, merely by adding hot water is capable - in the range of 80-100°C, of forming aqueous drink and in which the anti-inflammatory drugs (NSAIDs), ibuprofen include the alkali metal salts such as the sodium and potassium salts; the amino acid salts such as the lysine or arginine salts, or amine salts such as the meglumine salt or a pharmaceutically acceptable salt thereof can be maintained as a discrete phase when in the liquid state minimising the need for extra steps such as stirring. Thus, lets to avoid any waiting time.

ibuprofen include the alkali metal salts such as the sodium and potassium salts; the amino acid salts such as the lysine or arginine salts, or amine salts such as the meglumine salt or a pharmaceutically acceptable salt thereof ibuprofen in combination with decongestant in the form of the power or granules for preparation hot solution as a hot drink is used to relieve symptoms of colds and flu, such as: pain with fever, cramps in the nose and sinuses, cough.

There is no disclosure of how to formulate a simple ibuprofen include the alkali metal salts such as the sodium and potassium salts; the amino acid salts such as the lysine or arginine salts, or amine salts such as the meglumine salt or a pharmaceutically acceptable salt thereof composition which can be used to provide a stable aqueous preparation on the addition of hot water and maintain ibuprofen as a discrete phase taking into account its melting point characteristics.

ibuprofen lysine (the lysine salt of ibuprofen) can be referred to interchangeable as ibuprofen lysinate, ibuprofen (L-lysine), p-lsobutylphenyl-2-propionate lysine, L-Lysine mono(4-isobutyl-alpha-methylbenzeneacetate), α-methyl-4-(2-methyl propyl) benzeneacetic acid lysine salt, L-lysine compound with 2-(4-isobutylphenyl) propanoic acid (1:1) and ibuprofen D, L- lysine.

Composition containing ibuprofen are usually provided in unit doses give 200 mg, 400 mg, 600 mg, or 800 mg ibuprofen.

Adult and children 12 years or older dose for fever, initial dose - 200 mg orally every 4 to 6 hours, may increase to 400 mg every 4 to 6 hours if needed. Maximum dose: 1200 mg/day. Adult and children 12 years or older dose for pain - 200 to 400 mg orally every 4 to 6 hours as needed. Maximum dose1200 mg/day for over the counter (OTC).

As second active ingredient is used decongestant selected from the group pseudoephedrine, phenylpropanolamine and phenylephrine or a pharmaceutically acceptable salt thereof.

Pseudoephedrine or phenylephrine or a pharmaceutically acceptable salt thereof is used to relieve nasal discomfort caused by colds, allergies, and hay fever. It is also used to relieve sinus congestion and pressure. Phenylephrine will relieve symptoms but will not treat the cause of the symptoms or speed recovery. Phenylephrine is in a class of medications called nasal decongestants. It works by reducing swelling of the blood vessels in the nasal passages.

Phenylephrine is commonly used as a nasal decongestant to help relieve a blocked nose. Adult and children 12 years or older dose for nasal congestion - 10 mg orally every 4 hours. Maximum dose: 60 mg per 24 hours

The usual adult dose of pseudoephedrine is 60 mg orally every 4-6 hours, up to a maximum of 240 mg per day. The usual paediatric dose of pseudoephedrine 15 mg orally every 6 hours, up to a maximum of 60 mg per day for ages 2-5 and 30 mg every 6 hours, up to a maximum of 120 mg per day for ages 6-12.

It is desired to formulate a composition containing non-steroidal anti-inflammatory drugs (NSAIDs) and decongestant which on addition of hot water forms hot aqueous drink for ingestion by patient. Such products have utility in the treatment of pain and the treatment of coughs and colds. The problem is to provide an easily formulated non-steroidal anti-inflammatory drugs (NSAIDs) and decongestant which forms a satisfactory drink product solely by the addition of hot water without requiring extra steps to be taken by the patient before adding hot water (in the range of 80-100°C) to non-steroidal anti-inflammatory drugs (NSAIDs) and decongestant composition.

Advantages achieved by the present invention are the provision of a composition that can be easily formulated by the manufacturer and an acceptably tasting drink product that can be prepared very easily by the patient together with the use of minimal quantities of homogenising excipients. It is especially advantageous that a hot drink can be prepared merely by adding hot water to the ibuprofen containing composition in the form of powder or granules.

The quantity of water to be added to the ibuprofen salt containing composition is therefore such as to provide an acceptable aqueous drink preparation. The lower amount of the quantity of water is usually determined by the dosage of ibuprofen salt containing composition, for example a low dosage such as 200mg requires less water to form a uniform dispersion or solution than a dose of 400mg ibuprofen. The upper end of the range is determined by the quantity of water that a person is prepared to drink for a single unit dose. Generally, it is anticipated that the amount of water will be in the range 10 ml-500ml, more preferably 50-300 ml, most preferably 100-250 ml.

The amount of the ibuprofen salt containing medicament in the ibuprofen composition will depend on the required treatment regimen. Administration of the aqueous preparation obtained from the ibuprofen composition may only be once daily or it may be several times daily, for example 2-4 times daily, for as long as the treatment is required. Each dosage unit of the ibuprofen salt containing composition suitably contains the ibuprofen medicament in an amount to give an equivalent dosage of 50-800mg ibuprofen, preferably 100-400mg and most preferably 200-400 mg ibuprofen per unit dose.

In a preferred embodiment of the pharmaceutical composition comprising combination of active ingredients an non-steroidal anti-inflammatory drugs (NSAIDs) selected from the salts of ibuprofen include the alkali metal salts such as the sodium and potassium salts; the amino acid salts such as the lysine or arginine salts, or amine salts such as the meglumine salt or a pharmaceutically acceptable salt thereof and of an effective amount decongestant selected from the group pseudoephedrine, phenylpropanolamine and phenylephrine or a pharmaceutically acceptable salt. More preferably, a non-steroidal anti-inflammatory drug (NSAIDs) is ibuprofen lysinate and decongestant is pseudoephedrine or phenylephrine. In the pharmaceutical composition, an non-steroidal anti-inflammatory drugs (NSAIDs) selected from the salts of ibuprofen include the alkali metal salts such as the sodium and potassium salts; the amino acid salts such as the lysine or arginine salts, or amine salts such as the meglumine salt or a pharmaceutically acceptable salt thereof is present in an amount from 7-85% by weight of the total weight of the composition and decongestant selected from the group pseudoephedrine, phenylpropanolamine and phenylephrine or a pharmaceutically acceptable salt is present in an amount from 0,1-9% by weight of the total weight of the composition.

in a preferred embodiment, the pharmaceutical composition comprises sweetener.

Preferably, the non-nutritive sweetener is selected from neotame, saccharin, sucralose, acesulfame-K, advantame, aspartame, cyclamate, stevia, monk fruit and nutritive sweetener sucrose, fructose, dextrose, and maltose. More preferably, the sweetener is stevia or sucrose. In the pharmaceutical composition, sweetener is present in an amount from 0.06 to 0.8 % by weight of the total weight of the composition.

In a preferred embodiment, the active agent is micronized, preferably, the active agent is micronized. In a preferred embodiment, the active agent has a particle size volume distribution with a D50 under 40 microns and a D90 under 80 microns, preferably the active agent has a particle size volume distribution with a D50 under 25 microns and a D90 under 60 microns, more preferably, the active agent has a particle size volume distribution with a D50 under 15 microns and a D90 under 20 microns. In a preferred embodiment, the active agent has a particle size volume distribution with a D50 between 1 and 15 microns and a D90 between 3 and 20 microns.

In a preferred embodiment, the pharmaceutical composition comprises flavour. Preferably, the flavour is lemon, lemon/orange, tangerine, raspberry, sambucus nigra and forest berries. More preferably, the flavour is lemon. In the pharmaceutical composition, flavour is present in an amount from 0.1 to 1.5% by weight of the total weight of the composition.

in a preferred embodiment, the pharmaceutical composition comprises at least a lubricant. Preferably, the lubricant is selected from polyethylene glycol, magnesium stearate, sodium stearyl fumarate, calcium stearate, stearic acid, and mixtures thereof. More preferably, the lubricant is sodium stearyl fumarate. In the pharmaceutical composition, lubricant is present in an amount from 0.06 to 0.8 % by weight of the total weight of the composition.

in a preferred embodiment, the pharmaceutical composition comprises at least a filler. Preferably, the filler is selected from starch, sugar, dextrin, lactose, compressible starch, microcrystalline cellulose (MCC), inorganic salts (mainly inorganic calcium salt, calcium sulphate), mannitol. Preferably, the glidant is a mannitol. In the pharmaceutical composition, filler is present in an amount from 4 to 50% by weight of the total weight of the composition.

In a preferred embodiment, the pharmaceutical composition comprises at least an acidifier / taste improver. Preferably, the acidifier / taste improver is selected from citric acid or citric acid monohydrate, sodium citrate and mixtures thereof. More preferably, the acidifier / taste improver is citric acid. In the pharmaceutical composition, acidifier / taste improver is present in an amount from 0.1 to 1.3 % by weight of the total weight of the composition.

In another preferred embodiment of the pharmaceutical composition as disclosed herein, the pharmaceutical composition consists essentially of a non-steroidal anti-inflammatory drug (NSAIDs) is ibuprofen lysinate and decongestant is pseudoephedrine or phenylephrine. In the pharmaceutical composition, an non-steroidal anti-inflammatory drugs (NSAIDs) selected from the salts of ibuprofen include the alkali metal salts such as the sodium and potassium salts; the amino acid salts such as the lysine or arginine salts, or amine salts such as the meglumine salt or a pharmaceutically acceptable salt thereof in the form of powder or granules for making an aqueous solution with water at a temperature in the range 80-100°C, having a particle size volume distribution with a D50 under 15 microns and a D90 under 20 microns and at least one pharmaceutically acceptable excipient, wherein the pharmaceutical composition is prepared by dry granulation.

As used herein, the term "granulate" refers to a population of granules. The granules as disclosed herein, can be prepared by any granulation method. As used herein, the term "granulation" refers to the process of agglomerating powder particles into larger agglomerates (i.e., granules) that contain the active substances. The term "granulation" includes dry and wet granulation techniques. The term "wet granulation" refers to any process comprising the steps of addition of a liquid to the powder starting materials, preferably kneading, and drying to yield a solid dosage form. The term "dry granulation" refers to any process that comprises compacting the powder, usually either by slugging or with a roller compactor, and preferably milling the compacted powder to obtain the granules. No liquid is employed for the dry granulation. The compacted granulate or compacted granules as disclosed herein are prepared by dry granulation.

In a preferred embodiment, it has also been found the use of a granulation process to product pharmaceutical composition, rather than a direct compression process, results in improved flow and compressibility properties of ibuprofen lysinate. Both wet and dry granulation process improved compressibility. However, unexpectedly, when the ibuprofen lysinate granulation the stability of the ibuprofen lysinate was unsatisfactory; on contrary by using the dry granulation process fulfilled all stability issues. Preferably, the pharmaceutical composition is prepared by dry granulation. More preferably, the pharmaceutical composition comprises compacted granules containing the active agent.

in a preferred embodiment of the first aspect of the present invention, the pharmaceutical composition to form granules or a granulated mixture. The granules or granulated mixture filled into sachets to form solid oral dosage forms.

The invention also describes a process for the manufacture of the powder or granules.

A pharmaceutical composition in the form of powder or granules for making an aqueous solution with water at a temperature in the range 80-100°C comprises following components in the following percentages by weight:
- from 7-85% of ibuprofen include the alkali metal salts such as the sodium and potassium salts; the amino acid salts such as the lysine or arginine salts, or amine salts such as the meglumine salt or a pharmaceutically acceptable salt thereof.
- from 0,1-9% decongestant selected from the group pseudoephedrine, phenylpropanolamine and phenylephrine or a pharmaceutically acceptable salt thereof.
- from 0.1-1.3 % acidifier / taste improver
- from 4-50 % fillers
- from 0.06-0.8 % sweetener (non-nutritive or nutritive)
- from 0.1-1.5 % flavour and colorant

Additionally, the pharmaceutical composition in the form of powder or granules for making an aqueous solution with water at a temperature in the range 80-100°C could contain 0.06-0.8% of lubricant.

Specifically, the pharmaceutical composition in the form of powder or granules for making an aqueous solution with water at a temperature in the range 80-100°C comprises following components in the following percentages by weight:
- from 7-85% ibuprofen lysinate
- from 0,7-9% pseudoephedrine hydrochloride
- from 0.1-1.3 % citric acid
- from 4-50 % mannitol
- from 0.06-0.8 5% of sucrose
- from 0.1-1.5 % flavour and colorant

Additionally, the pharmaceutical composition in the form of powder or granules for making an aqueous solution with water at a temperature in the range 80-100°C could contain 0.1-1.3 % sodium citrate and 0.06-0.8% of sodium stearyl fumarate.

Specifically, the pharmaceutical composition in the form of powder or granules for making an aqueous solution with water at a temperature in the range 80-100°C comprises following components in the following percentages by weight:
- from 7-85% ibuprofen lysinate
- from 0.1-1.5% phenylephrine hydrochloride
- from 0.1-1.3 % citric acid
- from 4-50 % mannitol
- from 0.06-0.8 5% of sucrose
- from 0.1-1.5 % flavour and colorant

Additionally, the pharmaceutical composition in the form of powder or granules for making an aqueous solution with water at a temperature in the range 80-100°C could contain 0.1-1.3 % sodium citrate and 0.06-0.8% of sodium stearyl fumarate.

Dose form of the product: powder 200 mg or 400 mg of ibuprofen (as lysine) powder in the fixed combination of 30 mg or 60 mg pseudoephedrine (as hydrochloride).

Dose form of the product: powder 200 mg or 400 mg of ibuprofen (as lysine) powder in the fixed combination of 5 mg or 10 mg phenylephrine (as hydrochloride).

The recommended daily dose of ibuprofen is not more than 1200 mg, and this product will be in packs containing not more than 12 dose units when sold in the manufacturer's original pack. Packs will be labelled for use by adults and children over 12 years of age.

The hot drink formulation is preferred will decrease the need for consumers to seek additional or alternative forms or doses of pain relief and decrease the risk of double dosing with other products or formulations.

The product is practical and easy to use. The product is in powder form and is to be dissolved in hot (not boiling >95°C) water and consumed immediately.

In another aspect, for combination ibuprofen pseudoephedrine oral powders or granules for constitution can be prepared by combining the components of the mixture and dry blending, in a bin blender or with dry granulation.

The present invention relates to a process for the manufacture of the pharmaceutical composition comprising at least an active agent, wherein the active agent is a non-steroidal anti-inflammatory drug (NSAIDs) is ibuprofen lysinate and decongestant is pseudoephedrine or phenylephrine. In the pharmaceutical composition, a non-steroidal anti-inflammatory drug (NSAIDs) selected from the salts of ibuprofen include the alkali metal salts such as the sodium and potassium salts; the amino acid salts such as the lysine or arginine salts, or amine salts such as the meglumine salt or a pharmaceutically acceptable salt thereof, comprising the following steps:
a) weighing, sieving, and mixing the active agent and optionally at least one pharmaceutically acceptable excipient,
b) granulating the mix of step, a),
c) optionally mixing the granules of step b) with at least one pharmaceutically acceptable excipient, and
d) mixing the granules obtained in step b).

The term dry granulation refers to any process that comprises compacting the powder, usually either by slugging or with a roller compactor, and preferably milling the compacted powder to obtain the granules. No liquid is employed for the dry granulation. The compacted granulate or compacted granules as disclosed herein are prepared by dry granulation.

In another aspect, the present invention relates to the pharmaceutical composition manufactured according to the processes as disclosed herein. Another aspect of the present invention relates to the pharmaceutical composition of the previous aspect for use in the treatment of inflammation, pain, fever and/or cold-like symptoms, like nasal congestion in a mammalian organism and adapted for unit dosage oral administration.

The following examples more particularly illustrate the present invention by referring to the following non-limiting examples.

### EXAMPLE 1

Qualitative and quantitative pharmaceutical composition in the form of powder or granule form are made as per the formula below:

| | Ingredient | Per cent, %, w/w | Per strength, mg | | Function |
|---|---|---|---|---|---|
| | | | Per sachet 200/30 mg | Per sachet 400/60 mg | |
| 1. | Ibuprofen lysine (eq. to ibuprofen) | 56.92 | 341.74* | 683.48* | active substance |
| 2. | Pseudoephedrine hydrochloride (eq. to pseudoephedrine) | 6.10 | 36.62^{#} | 73.24^{##} | active substance |
| 3. | Flavour and colorant^ | 1.00 | 6 | 12 | Flavour and colorant |
| 4. | Citric acid (hydrate) | 0.83 | 5 | 10 | Acidifier / taste improver |
| 5. | Sodium citrate (with or without) | 0.83 | 5 | 10 | Acidifier / taste improver |
| 6. | Mannitol | 33.31 | 200 | 400 | Filler |
| 7. | Stevia or Sucrose | 0.50 | 3 | 6 | Sweetener (Non-Nutritive or Nutritive) |
| 8. | Sodium stearyl fumarate | 0.50 | 3 | 6 | Lubricant (on demand) |
| | Total: | 100.00 | 600.36 | 1200.72 | |

| | | | | | |
|---|---|---|---|---|---|
| *Equivalent to 200 mg ibuprofen **Equivalent to 400 mg ibuprofen # Equivalent to 30 mg pseudoephedrine ## Equivalent to 60 mg pseudoephedrine ^ Flavor and colorant defines | | | | | |

| | |
|---|---|
| *Lemon* | *Yellow* |
| *Lemon* / *Orange* | *Yellow* / *orange* |
| *Tangerine* | *Orange* |
| *Raspberry* | *Pink* |
| *Sambucus Nigra* | *Dark red* |
| *Forest berries* | *Dark red* |

### EXAMPLE 2

Qualitative and quantitative pharmaceutical composition in the form of powder or granule form are made as per the formula below:

| | Ingredient | Per cent, %, w/w | Per strength, mg | | Function |
|---|---|---|---|---|---|
| | | | Per sachet 200/5 mg | Per sachet 400/10 mg | |
| 1. | Ibuprofen lysine (eq. to ibuprofen) | 56.92 | 341.74* | 683.48* | active substance |
| 2. | Phenylephrine hydrochloride (eq. to phenylephrine) | 1.07 | 6.09 | 12.18 | active substance |
| 3. | Flavour and colorant^ | 1.05 | 6 | 12 | Flavour and colorant |
| 4. | Colorant | Avoid any azo dyes (Children population) | | | Colorant |
| 5. | Citric acid (hydrate) | 0.88 | 5 | 10 | Acidifier/taste improver |
| 6. | Sodium citrate (with or without) | 0.88 | 5 | 10 | Acidifier/taste improver |
| 7. | Mannitol | 35.10 | 200 | 400 | Filler |
| 8. | Stevia or Sucrose | 0.53 | 3 | 6 | Sweetener (Non-Nutritive or Nutritive) |
| 9. | Sodium stearyl fumarate | 0.53 | 3 | 6 | Lubricant (on demand) |
| | Total: | 100.00 | 569.83 | 1139.66 | |

| | | | | | |
|---|---|---|---|---|---|
| *Equivalent to 200 mg ibuprofen **Equivalent to 400 mg ibuprofen # Equivalent to 5 mg phenylephrine ## Equivalent to 10 mg phenylephrine ^ Flavour and colorant defines | | | | | |

| | |
|---|---|
| *Lemon* | *Yellow* |
| *Lemon* / *Orange* | *Yellow* / *orange* |
| *Tangerine* | *Orange* |
| *Raspberry* | *Pink* |
| *Sambucus Nigra* | *Dark red* |
| *Forest berries* | *Dark red* |

The granular mixture of EXAMPLE 1 or EXAMPLE 2 is packed into sachets to yield 200 mg or 400 mg ibuprofen per sachet.

The ibuprofen lysine and pseudoephedrine hydrochloride or phenylephrine hydrochloride pharmaceutical composition in the form of powder or granules are used to fill the final unit single-dose package for subsequent oral administration of the product after dissolving in hot water, which must provide light and moisture protection for the powder or granules. One example of suitable packaging is foil (for example aluminium) pouch or sachet. Additionally laminated polyethylene terephthalate (PET) aluminium foil or laminated aluminium paper foil, which offer good protection against moisture and light. The aluminium layer present in both laminated aluminium paper foil and laminated PET aluminium foil offers a similar level of protection from sunlight and moisture. Sachets of laminated material (paper/polyethylene film/aluminium foil/polyethylene film) or sachets of laminated material (paper/polyethylene film/aluminium foil/ethylene and acrylic acid copolymer).

Smaller quantities of hot water (in the range 80-100 °C) such as 150 ml, 100 ml and 50 ml may be added to the granular mixture of EXAMPLE 1 or EXAMPLE 2 to form a solution/dispersion.

The contents of the sachet were placed into a cup or glass and 150-200 ml hot water (in the range 80-100 °C) added and stir until dissolved, thoroughly until the powder or granules dissolves.

Manufacture of the pharmaceutical composition of ibuprofen lysine and pseudoephedrine hydrochloride or phenylephrine hydrochloride in the form of powder or granules by dry granulation.

All ingredients were sifted through a 0.8-1.2 mm sieve. The ibuprofen lysine was mixed with pseudoephedrine hydrochloride. The dry mix was then compacted either by slugging or with a roller compactor. The compacted mix was milled and sieved. The granulate were sift through 0.6-0.8 mm sieve. Furthermore lubricant - Sodium stearyl fumarate was added and mixed with the compacted granule. The lubricated granule was filled into pouch or sachet The lubricated granule was filled into pouch or sachet.

### EXAMPLE 3

### Stability

Pharmaceutical composition of EXAMPLE 1 and EXAMPLE 2 were subjected tests at times 0, 3 months and 6 months at 40°C and 75% Relative Humidity (RH)

The total impurities are shown below.

| Impurity % | Ex.6 0 months | Ex.6 3 months | Ex.6 6 months |
|---|---|---|---|
| 40°C/75% RH | <0.05 % | <0.05 % | <0.05 % |

### EXAMPLE 4

### Water content

The amount of water of the pharmaceutical composition of Examples 1 to 2. The water content of the pharmaceutical composition of Examples 1 to 2 was about 3%.

Unless otherwise indicated, all analysis methods were carried out according to the European Pharmacopoeia 11^{th} edition.

## Claims

1. A pharmaceutical composition comprising combination of an non-steroidal anti-inflammatory drugs (NSAIDs) selected from the salts of ibuprofen include the alkali metal salts such as the sodium and potassium salts; the amino acid salts such as the lysine or arginine salts, or amine salts such as the meglumine salt or a pharmaceutically acceptable salt thereof and of an effective amount decongestant selected from the group pseudoephedrine, phenylpropanolamine and phenylephrine or a pharmaceutically acceptable salt thereof in the form of powder or granules for making a solution with water at a temperature in the range 80-100°C.

2. Pharmaceutical composition according to claim 1 wherein a non-steroidal anti-inflammatory drug (NSAIDs) is ibuprofen lysine and wherein decongestant is pseudoephedrine hydrochloride.

3. Pharmaceutical composition according to claim 1 wherein a non-steroidal anti-inflammatory drug (NSAIDs) is ibuprofen lysine and wherein decongestant is phenylephrine hydrochloride.

4. Pharmaceutical composition according to claim 2, wherein ibuprofen lysine is in the range 7-85% and pseudoephedrine hydrochloride is 0.7-9%.

5. Pharmaceutical composition according to claim 2, wherein ibuprofen lysine is in the range 7-85% and phenylephrine hydrochloride is 0.1-1.5%.

6. Pharmaceutical composition according to claim 1 in the form of powder or granules for making an aqueous solution with water at a temperature in the range 80-100°C, wherein the pharmaceutical composition is prepared by dry granulation.

7. Pharmaceutical composition according to claims 1 to 5 for use in the treatment of inflammation, pain, fever and/or cold-like symptoms, like nasal congestion in a mammalian organism and adapted for unit dosage oral administration.

8. A pharmaceutical composition in the form of powder or granules for making an aqueous solution with water at a temperature in the range 80-100°C comprises following components in the following percentages by weight:
• from 7-85% of ibuprofen include the alkali metal salts such as the sodium and potassium salts; the amino acid salts such as the lysine or arginine salts, or amine salts such as the meglumine salt or a pharmaceutically acceptable salt thereof.
• from 0,1-9% decongestant selected from the group pseudoephedrine, phenylpropanolamine and phenylephrine or a pharmaceutically acceptable salt thereof.
• from 0.1-1.3 % acidifier / taste improver
• from 4-50 % fillers
• from 0.06-0.8 % sweetener (non-nutritive or nutritive)
• from 0.1-1.5 % flavour and colorant

9. Pharmaceutical composition according to claim 8, wherein pharmaceutical composition further contains 0.06-0.8 % of lubricant.

10. A pharmaceutical composition in the form of powder or granules for making an aqueous solution with water at a temperature in the range 80-100°C comprises following components in the following percentages by weight:
• from 7-85% ibuprofen lysinate
• from 0,7-9% pseudoephedrine hydrochloride
• from 0.1-1.3 % citric acid
• from 4-50 % mannitol
• from 0.06-0.8 % of sucrose
• from 0.1-1.5 % flavour and colorant

11. Pharmaceutical composition according to claim 10, wherein pharmaceutical composition further contains 0.1-1.3 % sodium citrate and 0.06-0.8% of sodium stearyl fumarate.

12. A pharmaceutical composition in the form of powder or granules for making an aqueous solution with water at a temperature in the range 80-100°C comprises following components in the following percentages by weight:
• from 7-85% ibuprofen lysinate
• from 0.1-1.5% phenylephrine hydrochloride
• from 0.1-1.3 % citric acid
• from 4-50 % mannitol
• from 0.06-0.8 5% of sucrose
• from 0.1-1.5 % flavour and colorant

13. Pharmaceutical composition according to claim 12, wherein pharmaceutical composition further contains 0.1-1.3 % sodium citrate and 0.06-0.8 % of sodium stearyl fumarate.
